# EUROPEAN PATENT SPECIFICATION

(11) **EP 3 000 383 B1**
(45) Date of publication and mention of the grant of the patent: **11.12.2019**
(21) Application number: 14185921.5
(22) Date of filing: 23.09.2014
(51) Int. Cl.: A61B 3/113, A61B 5/11, A61B 5/00

(54) **Head mountable device for measuring eye movement**
Am Kopf montierbare Vorrichtung zur Messung der Augenbewegung
Dispositif pouvant être porté sur la tête pour la mesure de mouvement oculaire

(43) Date of publication of application: 30.03.2016
(73) Proprietor: Natus Medical Incorporated, Pleasanton, CA 94566 (US)
(72) Inventor: Macdougall, Hamish, Woolloomooloo, New South Wales 2011 (AU); Weber, Konrad P., 8006 Zürich (CH); Andersen, Anders Thøsing, 4623 Lille Skensved (DK); Kopilewicz, Izaak, 2700 Brønshøj (DK)
(74) Representative: Zacco Denmark A/S

(56) References cited:
- US-A- 5 094 521
- US-A- 5 751 258
- US-A- 5 838 420
- US-A1- 2004 181 168
- A H Clarke ET AL: "Using high frame rate CMOS sensors for three-dimensional eye tracking", Behavior research methods, instruments, & computers : a journal of the Psychonomic Society, Inc, 1 November 2002 (2002-11-01), pages 549-560, XP055136469, United States DOI: 10.3758/BF03195484 Retrieved from the Internet: URL:http://www.ncbi.nlm.nih.gov/pubmed/125 64559

## Description

### FIELD

The present disclosure relates to a device for measuring eye movement, in particular a head mountable device for measuring eye movement in relation to tests involving deprivation of sight of one or more of the eyes of a user. Such tests may be ophthalmologic, vestibular, and/or neurologic tests.

### BACKGROUND

There is an ongoing investigation towards developing measurement techniques and equipment for measuring eye movement of humans. Various ophthalmologic, vestibular and neurologic tests exists which involves observing eye movements. Tests may comprise observation of eye movements during concurrent sight deprivation of either one eye or both eyes. Tests incorporating deprivation of sight of either one or both eyes may comprise HINTS tests, or individual parts of the HINTS tests, such as test of skew and/or tests of nystagmus.

Tests may comprise measuring fast eye movements, e.g. eye saccades, lasting approximately between 20-200 ms and involving angular speed up to 900 deg/s. Such fast movements may be visual to the clinician, but may be difficult to quantify consistently. Tests may comprise measuring very small eye deviations, e.g. skew, which may be difficult to detect and/or quantify subjectively.

It is desirable to circumvent subjective measurements and provide a possible standardized test, which is independent of the clinician or other person performing the test. Furthermore, in some environments, such as in pre hospital settings, it may be problematic, if not impossible, to accurately perform the test when relying on subjective measurements.

Furthermore, sight deprivation is conventionally performed by placing a black cover over the patient's eye. The cover may be a hard plastic or the hand of the clinician. Some devices may comprise a black cover, such as a visor, which is able to be lowered over the patient's eyes. However, it is desirable that a device capable of performing the above mentioned tests is able to control the deprivation of sight in an accurate, consistent, secure, comfortable, and easy way.

US5838420A relates to a method and apparatus for ocular motility testing, and discloses an apparatus for ocular motility testing comprising, an item of headgear onto which is mounted: gaze-detection means for determining the visual axis directions of one or both eyes of an observer wearing the headgear in relation to a reference primary position; and projector means for projecting a target onto a screen spaced from the observer.

US5094521A discloses an apparatus for evaluating the alignment of both eyes of a patient objectively in primary, secondary and tertiary gaze, and the Bielschowsky head tilt positions and to diagnose for phorias, tropias and intermittent tropias. The apparatus has a head restraint and a mounted fixation target in a spaced relationship to the head restraint and having adjustments for adjusting the target along three axis. Electronic shutters are used to occlude each eye during the testing. A camera is used for imaging the eye of a patient having his head in the head restraint with his eyes aligned with the target. The camera is mounted adjacent to the target on the xyz mount. A microcomputer has a monitor screen, and is operatively coupled to the camera for receiving a series of images from the camera stored data for each frame, and comparing one frame image with another frame image of the same eye and gaze position or both eyes in the same frame to thereby determine a patient's ocular alignment, pupil size, corneal diameter and interpupillary distance.

US5751258A relates to a liquid crystal lens driver electronics for eye protection, high speed shuttering with consistent performance, and discloses a power supply circuit for a liquid crystal welding lens or shutter which provides a consistent and stable regulated power signal for driving or powering the liquid crystal shutter for maintaining stable performance over the life of the power supply and a battery level indicator feature to provide advance warning of degraded device performance.

### SUMMARY

There is a need for an improved device which avoids the use of subjective measures in ophthalmologic, vestibular, and/or neurologic tests, and which avoid or limits the need of user interaction during tests, and hence is able to reliably measure eye movement when performing various tests. The present disclosure provides a device and a method which provides objective and reproducible measurement of eye movement in tests requiring deprivation of sight.

Disclosed is a head mountable device for measuring eye movement. The head mountable device comprises a frame, a camera system, and a first liquid crystal display (LCD) shutter. The camera system comprises a first camera, wherein the camera system is configured to obtain a first set of images of a first eye of a user. The first set of images is configured to be obtained with a first frame rate, wherein the first frame rate is selected such as to enable detection of eye saccades of the first eye. The first liquid crystal display (LCD) shutter is configured to control passage of light to at least part of the first eye based on a first control signal. The first LCD shutter is configured to operate in a first primary mode and a first secondary mode, where passage of light through the first LCD shutter in the first secondary mode is restricted relative to the first primary mode. The head mountable device further comprises a second liquid crystal display (LCD) shutter configured to control passage of light to at least part of a second eye of the user based on a second control signal. The second LCD shutter is configured to operate in a second primary mode and a second secondary mode, where passage of light through the second LCD shutter in the second secondary mode is restricted relative to the second primary mode. The first LCD shutter is configured to control passage of light through the first LCD shutter based on the first control signal and a common control signal, and the second LCD shutter is configured to control passage of light through the second LCD shutter based on the second control signal and said common control signal. The first control signal, the second control signal and the common control signal are voltage signals.

Also disclosed is a method for measuring eye movement of a user using a head mountable device, such as the disclosed head mountable device, comprising a frame, a camera system comprising a first camera, and a first liquid crystal display (LCD) shutter configured to control passage of light to at least part of a first eye of a user based on a first control signal. The first LCD shutter is configured to operate in a first primary mode and a first secondary mode, where passage of light through the first LCD shutter in the first secondary mode is restricted relative to the first primary mode. The head mountable device further comprises a second liquid crystal display (LCD) shutter configured to control passage of light to at least part of a second eye of the user based on a second control signal. The second LCD shutter is configured to operate in a second primary mode and a second secondary mode, where passage of light through the second LCD shutter in the second secondary mode is restricted relative to the second primary mode. The method comprising:
- adjusting passage of light to at least part of the first eye by operation of the first LCD shutter;
- obtaining a first set of images of the first eye by the camera system, wherein the first set of images is configured to be obtained with a first frame rate, wherein the first frame rate is selected such as to enable detection of eye saccades of the first eye;

- adjusting passage of light to at least part of the second eye by operation of the second LCD shutter;
- obtaining a second set of images of the second eye by the camera system;
- configuring the first LCD shutter to control passage of light through the first LCD shutter based on the first control signal and a common control signal; and
- configuring the second LCD shutter to control passage of light through the second LCD shutter based on the second control signal and said common control signal, where the first control signal, the second control signal and the common control signal are voltage signals.

The method may be implemented with the device for measuring eye movement. At least a part of the method may be incorporated in software adapted to run in a processing unit, such as a processing unit of the device for measuring eye movement.

It is envisaged that any embodiments or elements as described in connection with any one aspect may be used with any other aspects or embodiments, mutatis mutandis.

The method and apparatus as disclosed enables fast and objective examination of ophthalmologic, vestibular and neurologic parameters. Objective examinations as an alternative to conventional subjective assessments may provide more reliable and consistent examinations. Hence, incorrect or unnecessary treatment may be avoided, and improved possibility of detecting changes in a patient's condition is provided. Furthermore, the disclosed method and apparatus provides automatically controlled sight deprivation, which enables automation and standardization of tests requiring sight deprivation, and/or limits the need for a clinician's interaction during the test. Furthermore, electronic control of the sight deprivation improves the accuracy and precision of measurements.

The head mountable device comprises a frame. The frame may be configured to be fixated to the head of the user, e.g. by adjustable and/or elastic straps. The frame may be in the form a goggle, a helmet, a cap, and/or another head mountable equipment. In an embodiment, the frame is embodied as a goggle. The frame may be configured to fasten the head mountable device to the head of the user such as to prevent motion of the head mountable device relative to the head of the user. The frame may accommodate elements of the head mountable device. The frame may accommodate the camera system and/or the first LCD shutter. The camera system and/or the first LCD shutter may be attached to the frame.

The method may further comprise mounting the head mountable device and/or the frame to the head of the user.

The head mountable device may be operable without attached wires. The head mountable device may comprise a power supply, such as a battery power supply and/or a power inlet. The frame may accommodate power supply. The power supply may be attached to the frame. Providing a power supply may allow operation of the head mountable device without the need of a power outlet, thus providing an increased scope of operation, e.g. the head mountable device may be used in an ambulance or at an accident site.

The head mountable device may be configured to control passage of light to at least part of a second eye of the user. For example, the first LCD shutter may additionally be configured to control passage of light to at least a part of the second eye. Alternatively and/or additionally, the head mountable device may comprise a second liquid crystal display (LCD) shutter configured to control passage of light to at least part of the second eye of the user based on a second control signal. The second LCD shutter may be configured to operate in a second primary mode and a second secondary mode, where passage of light through the second LCD shutter in the second secondary mode is restricted relative to the second primary mode. The frame may accommodate the second LCD shutter.

The first LCD shutter may be configured to allow less light to reach the first eye in the first secondary mode than in the first primary mode.

The second LCD shutter may be configured to allow less light to reach the second eye in the second secondary mode than in the second primary mode.

Passage of light through the first LCD shutter in the first primary mode, and/or passage of light though the second LCD shutter in the second primary mode, may be configured to allow passage of a certain amount of light. Passage of light through the first LCD shutter in the first primary mode, and/or passage of light though the second LCD shutter in the second primary mode, may be restricted relative to complete passage of light. For example, passage of light through the first LCD shutter in the first primary mode, and/or passage of light though the second LCD shutter in the second primary mode, may be restricted to less than 90 % of the light reaching the respective first and/or second LCD shutter, or less than 85 % of the light reaching the respective first and/or second LCD shutter, or less than 70 % of the light reaching the respective first and/or second LCD shutter, or less than 50 % of the light reaching the respective first and/or second LCD shutter, or less than 30 % of the light reaching the respective first and/or second LCD shutter.

Passage of light through the first LCD shutter in the first secondary mode, and/or passage of light though the second LCD shutter in the second secondary mode, may be configured to block passage of light. For example, passage of light through the first LCD shutter in the first secondary mode, and/or passage of light though the second LCD shutter in the second secondary mode, may be restricted to less than 5 % of the light reaching the respective first and/or second LCD shutter, or less than 10 % of the light reaching the respective first and/or second LCD shutter, or less than 20 % of the light reaching the respective first and/or second LCD shutter, or less than 35 % of the light reaching the respective first and/or second LCD shutter, or less than 50 % of the light reaching the respective first and/or second LCD shutter.

A common control signal may control passage of light through the first and/or second LCD shutter. The first LCD shutter may be configured to control passage of light through the first LCD shutter based on the first control signal and the common control signal. The second LCD shutter may be configured to control passage of light through the second LCD shutter based on the second control signal and the common control signal. For example, passage of light through the first LCD shutter and/or the second LCD shutter may be controlled by a phase difference between the common control signal and the first control signal and/or second control signal, respectively.

For example, the first control signal and the common control signal being out of phase, such as 180 degrees out of phase, or between 90 and 180 degrees out of phase, or having a phase difference of more than 90 degrees, may result in the first LCD shutter operating in the first secondary operating mode where passage of light through the first LCD shutter is restricted and/or blocked relative to the first primary operating mode. The first control signal and the common control signal being in phase, or having a phase difference of less than 90 degrees, may result in the first LCD shutter operating in the first primary operating mode where passage of light through the first LCD shutter is at least less restricted and/or blocked relative to the first secondary operating mode. Additionally and/or alternatively, the second control signal and the common control signal being out of phase, such as 180 degrees out of phase, or between 90 and 180 degrees out of phase, or having a phase difference of more than 90 degrees, may result in the second LCD shutter operating in the second secondary operating mode where passage of light through the second LCD shutter is restricted and/or blocked relative to the second primary operating mode. The second control signal and the common control signal being in phase, or having a phase difference of less than 90 degrees, may result in the second LCD shutter operating in the second primary operating mode where passage of light through the second LCD shutter is at least less restricted and/or blocked relative to the second secondary operating mode.

The control signals, such as the first control signal, the second control signal, and/or the common control signal, may be any types of signal, e.g. direct current (DC) and/or alternating current (AC) voltage signals. For example, the first control signal, the second control signal, and/or the common control signal may be alternating current (AC) signal(s). The first control signal, the second control signal, and/or the common control signal may be bipolar square wave voltage signal(s). It may be desirable to use a signal with an alternating component, such as AC signals and/or square wave signals, as this may prevent migration of crystals in the first and/or second LCD shutter(s), thereby decreasing the risk of damage to the first and/or second LCD shutter(s).

The voltage of the control signals, such as the first control signal, the second control signal, and/or the common control signal, may be chosen according to a specification of the LCD shutter(s). The voltage of the first control signal, the second control signal, and/or the common control signal may be in the range from 2 to 14 volts, such as 5 volts or such as 10 volts. The voltage difference between the first and/or second control signal(s) and the common control signal may be in the range from 2 to 14 volts, such as 5 volts or such as 10 volts.

In some tests, it may be beneficial to be able to obtain images of both eyes of a user. Hence, the camera system may be configured to obtain a second set of images of a second eye of the user. The first camera may be configured to obtain the first set of images and the second set of images. Alternatively and/or additionally, the camera system may comprise a second camera configured to obtain the second set of images.

The first set of images may be configured to be obtained with a first frame rate. The first frame rate may be selected such as to enable detection of eye saccades of the first eye. The second set of images may be configured to be obtained with a second frame rate. The second frame rate may be selected such as to enable detection of eye saccades of the second eye. The first frame rate and the second frame rate may be the same frame rate.

Obtaining the first set of images and/or the second set of images preferably enable detection of eye saccades of the first eye and/or of the second eye. Eye saccades may be very fast, e.g. eye saccades may last for only 20 ms. Therefore, the first frame rate and/or the second frame rate may be sufficiently high to enable reliable detection of eye saccades. For example, the first frame rate and/or the second frame rate may be higher than 125 frames per second (fps), such as higher than 150 fps, such as higher than 175 fps, such as higher than 200 fps, such as 250 fps. In other examples, the first frame rate and/or the second frame rate may be less than 125 fps, but is still sufficiently high to allow the processing unit to detect eye saccades of the first eye and/or of the second eye.

The head mountable device may comprise a first mirror for mirroring images of the first eye towards the first camera, and/or for mirroring images of the first eye towards the second camera, and/or for mirroring images of the second eye towards the first camera, and/or for mirroring images of the second eye towards the second camera. Additionally, the head mountable device may comprise a second mirror for mirroring images of the second eye towards the first camera and/or for mirroring images of the second eye towards the second camera.

The frame may accommodate the first mirror and/or the second mirror.

The first camera and/or the second camera may be focused on the first and/or second eye. The first camera and/or the second camera may be focused on the first and/or second eye, respectively, via the first and/or second mirror, respectively.

The head mountable device may comprise a first light source for emitting first electromagnetic radiation towards the first eye and/or the second eye. The first mirror and/or the second mirror may be configured to direct at least a part of the first electromagnetic radiation towards the first eye and/or the second eye.

The head mountable device may comprise a second light source for emitting second electromagnetic radiation towards the first and/or second eye. The first mirror and/or the second mirror may be configured to direct at least a part of the second electromagnetic radiation towards the first eye and/or the second eye.

The frame may accommodate the first light source and/or the second light source.

The first and/or second electromagnetic radiation may comprise infrared radiation, laser radiation, visible red radiation, visible blue radiation, visible green radiation, and/or visible orange radiation. The first and/or second electromagnetic radiation may comprise electromagnetic radiation with wavelengths in the range of 380-450 nm, or in the range of 450-495 nm, or in the range of 495-570 nm, or in the range of 570-590 nm, or in the range of 590-620 nm, or in the range of 620-750 nm, or in the range of 750-2.500 nm, or in the range of 2.500-10.000 nm, or in the range of 10.000-1.000.000 nm.

The first and/or second light source may be used for testing the first and/or second eye's response to light. The first and/or second light source may be used to light up the first and/or second eye. The first and/or second light source may be used to light up the first and/or second eye for the camera system to obtain images of the first and/or second eye.

The camera system and/or the first camera and/or the second camera may be configured to detect the first electromagnetic radiation and/or the second electromagnetic radiation.

The first and/or second mirror may be partly transparent. For example, the first and/or second mirror may be transparent to one or more selected ranges of electromagnetic radiation. The first and/or second mirror may be transparent to visible light, such as electromagnetic radiation with wavelengths in the range of 380-750 nm.

The first LCD shutter and/or the second LCD shutter may be configured to control passage of light with wavelengths of the first and/or second electromagnetic radiation.

The first LCD shutter and/or the second LCD shutter may be configured to allow passage of light with wavelengths of the first and/or second electromagnetic radiation. The first LCD shutter and/or the second LCD shutter may be configured to control passage of light with wavelengths different than the first and/or second electromagnetic radiation. For example, the first LCD shutter allows passage of infrared light in the first secondary mode, while passage of visible light through the first LCD shutter in the first secondary mode is restricted relative to the first primary mode. Additionally or in an alternative example, the second LCD shutter allows passage of infrared light in the second secondary mode, while passage of visible light through the second LCD shutter in the second secondary mode is restricted relative to the second primary mode.

The first LCD shutter may comprise a first polarizer having a first polarization. The second LCD shutter may comprise a second polarizer having a second polarization.

The first polarization may be different than the second polarization, e.g. the first polarization may be 90 degrees rotated relative to the second polarization.

The head mountable device may comprise one or more processing unit(s), such as a first processing unit and/or a second processing unit.

The head mountable device may comprise a processing unit, such as the first processing unit, configured to control the first LCD shutter and/or the second LCD shutter. The first control signal, the second control signal, and/or the common control signal may be controlled by the first processing unit.

The head mountable device may comprise a processing unit, such as the first processing unit or a second processing unit, configured to process the first set of images and/or the second set of images. The processing unit, e.g. the first processing unit and/or the second processing unit, may be configured to provide a processing unit output based on the first set of images and/or the second set of images.

The processing unit configured to process the first set of images may be the same processing unit as the processing unit configured to control the first LCD shutter and/or the second LCD shutter. For example, the first processing unit may be configured to process the first set of images and/or the second set of images and provide a processing unit output based on the first set of images and/or the second set of images, and the first control signal, the second control signal, and/or the common control signal may be controlled by the first processing unit.

The frame may accommodate the processing unit, such as the first processing unit and/or the second processing unit.

The head mountable device may comprise an interface for providing a device output based on the first set of images and/or the second set of images. The method may comprise providing a device output based on the first set of images and/or the second set of images. The interface may comprise one or more types of interfaces for providing the device output to a user and/or an operator of the head mountable device.

The frame may accommodate the interface.

The interface may comprise one or more display(s), such as a first display and/or a second display. The one or more display(s), such as the first display and/or the second display, may be an organic light emitting diode (OLED), an OLED display, a light emitting diode (LED), an LED display, and/or an e-ink display. The one or more display(s), such as the first display and/or the second display, may visually provide the device output, or part of the device output, to a user or an operator. The device output may comprise a visual output.

The interface may comprise one or more speaker(s), such as a first speaker and/or a second speaker. The one or more speaker(s), such as the first speaker and/or the second speaker, may audiologically provide the device output, or part of the device output, to a user or an operator. The device output may comprise an audiologic output.

The interface may comprise one or more wireless transmitter unit(s). The interface may comprise a wireless transceiver unit comprising the wireless transmitter unit and a wireless receiver unit. The wireless transmitter unit and/or the wireless transceiver unit and/or the wireless receiver unit may operate according to Bluetooth, WiFi, 3G, and/or 4G.

Providing the device output may comprise transmitting the device output wirelessly to an external display. The wireless transmitter unit may be configured to transmit the device output, or a part of the device output, to a display, such as an external display. The external display may be external to the head mountable device. The external display may be external to the frame of the head mountable device. The external display may be a display of a smartphone, a tablet computer, a laptop, a TV, a smart-TV, and/or the like.

The interface may comprise an input device for enabling control of the head mountable device, such as enabling control of the first LCD shutter and/or the second LCD shutter. The input device may enable control of the first LCD shutter and/or the second LCD shutter via control of the processing unit, such as the first processing unit. The input device may be the wireless receiver. Alternatively or additionally, the input device may comprise a touch display, a push button and/or a switch.

The head mountable device may comprise additional measurement units. For example, the head mountable device may comprise a motion sensor configured to detect movement of the head mountable device. The frame may accommodate the additional measurement units, such as the motion sensor. The motion sensor may comprise one or more gyroscope(s) and/or one or more accelerometer(s) and/or one or more camera(s). Additional measurement units may provide additional uses of the head mountable device, e.g. the head mountable device may be configurable to be used in more tests.

The frame may accommodate any or all of the above mentioned elements. Hence, the head mountable device may be configured as a standalone device without the need for external connections.

### BRIEF DESCRIPTION OF THE DRAWINGS

The above and other features and advantages will become readily apparent to those skilled in the art by the following detailed description of exemplary embodiments thereof with reference to the attached drawings, in which:
- Fig. 1: schematically illustrates an exemplary head mountable device,
- Fig. 2: schematically illustrates an exemplary head mountable device operating in a primary mode,
- Fig. 3: schematically illustrates an exemplary head mountable device operating in a secondary mode,
- Fig. 4: schematically illustrates an exemplary head mountable device,
- Fig. 5: schematically illustrates an exemplary head mountable device,
- Fig. 6: schematically illustrates an exemplary head mountable device,
- Fig. 7: schematically illustrates an exemplary camera system,
- Fig. 8: schematically illustrates an exemplary camera system,
- Fig. 9: schematically illustrates an exemplary head mountable device,
- Fig. 10: schematically illustrates an exemplary interface,
- Fig. 11: illustrates exemplary control signals for a head mountable device
- Fig. 12: shows a method for measuring eye movement.

### DETAILED DESCRIPTION

Various embodiments are described hereinafter with reference to the figures. Like reference numerals refer to like elements throughout. Like elements will, thus, not be described in detail with respect to the description of each figure. It should also be noted that the figures are only intended to facilitate the description of the embodiments. They are not intended as an exhaustive description of the claimed invention or as a limitation on the scope of the claimed invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout, the same reference numerals are used for identical or corresponding parts.

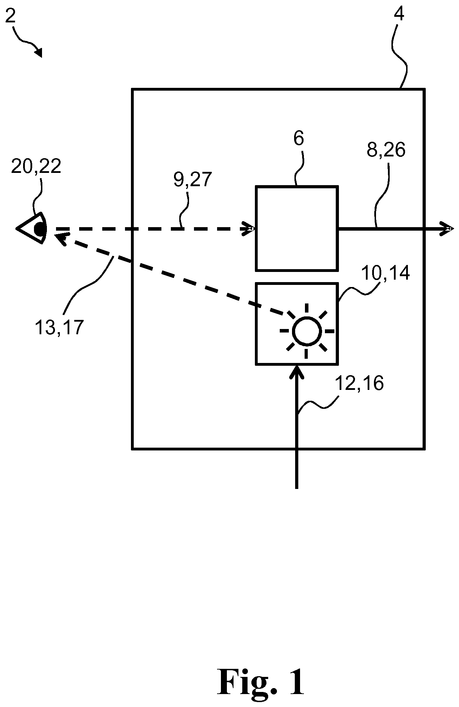

Fig. 1 schematically illustrates an exemplary head mountable device 2 for measuring eye movement. The head mountable device 2 comprises a frame 4, a camera system 6, and a first liquid crystal display (LCD) shutter 10. In the depicted example, the camera system 6 and the first LCD shutter 10 are mounted on the frame 4.

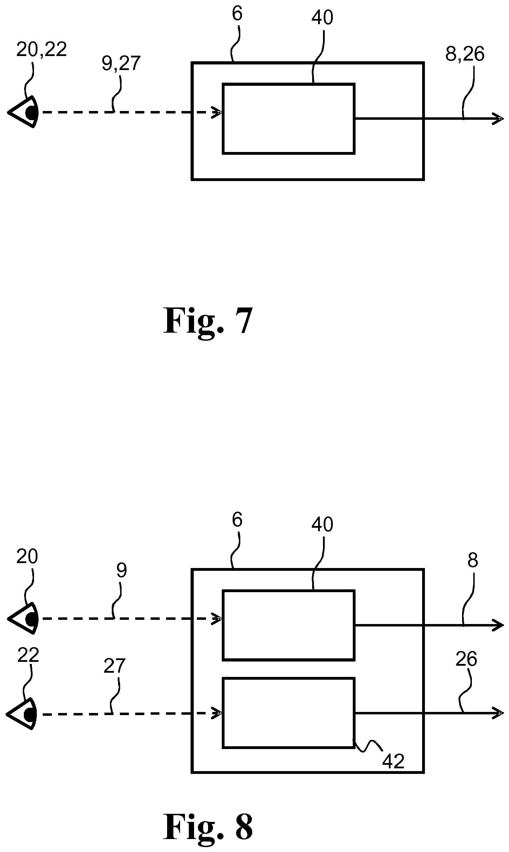

The camera system 6 comprises a first camera (Figs 7 and 8). The camera system 6 is configured to obtain a first set of images 8 of a first eye 20 of a user. Alternatively or additionally, the camera system 6 may be configured to obtain a second set of images 26 of a second eye 22 of the user. The camera system 6 detects images 9 of the first eye 20 and converts the images 9 of the first eye 20 to the first set of images 8 of the first eye 20. Alternatively or additionally, the camera system 6 detects images 27 of the second eye 22 and converts the images 27 of the second eye 22 to the second set of images 26 of the second eye 22.

The first LCD shutter 10 is configured to control passage of light 13 to at least part of the first eye 20 based on a first control signal 12. The first LCD shutter 10 is configured to operate in a first primary mode and a first secondary mode. Passage of light 13 through the first LCD shutter 10 in the first secondary mode is restricted relative to the first primary mode. For example, the first LCD shutter 10 may allow passage of light 13 when operating in the first primary mode, and block passage of light 13 when operating in the first secondary mode.

Alternatively or additionally the head mountable device 2 may comprise a second LCD shutter 14. The second LCD shutter 14 is configured to control passage of light 17 to at least part of the second eye 22 based on a second control signal 16. The second LCD shutter 14 is configured to operate in a second primary mode and a second secondary mode. Passage of light 17 through the second LCD shutter 14 in the second secondary mode is restricted relative to the second primary mode. For example, the second LCD shutter 14 may allow passage of light 17 when operating in the second primary mode, and block passage of light 17 when operating in the second secondary mode.

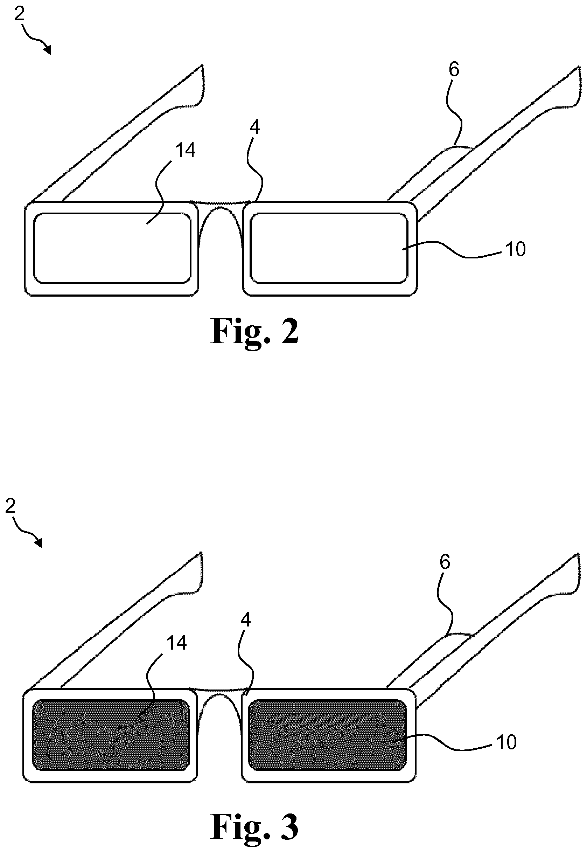

Fig. 2 schematically illustrates an exemplary head mountable device 2, wherein a first LCD shutter 10 is operating in a first primary mode, wherein passage of light 13 through the first LCD shutter 10 is allowed. Furthermore a second LCD shutter 14 is operating in a second primary mode, wherein passage of light 17 through the second LCD shutter 14 is allowed.

Fig. 3 schematically illustrates an exemplary head mountable device 2, wherein a first LCD shutter is operating in a first secondary mode, wherein passage of light 13 through the first LCD shutter 10 is restricted relative to the first primary mode as illustrated in Fig. 2. Furthermore a second LCD shutter 14 is operating in a second primary mode, wherein passage of light 17 through the second LCD shutter 14 is restricted relative to the second primary mode as illustrated in Fig. 2.

Figs. 2 and 3 illustrates examples wherein a first LCD shutter 10 and a second LCD shutter 14 are operating concurrently in a primary mode (Fig. 2) or in a secondary mode (Fig. 3). However, it is emphasized that the first LCD shutter 10 and the second LCD shutter 14 may operate independently. For example, the first LCD shutter 10 may be operating in the first primary mode while the second LCD shutter 14 is operating in the second primary mode.

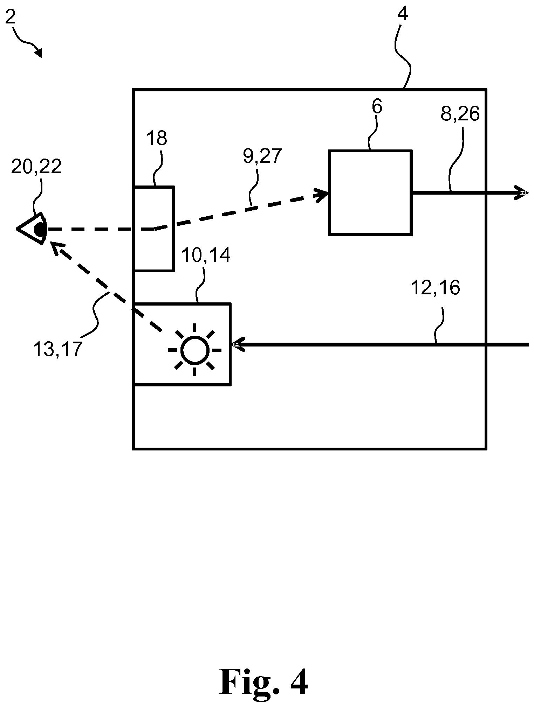

Fig. 4 schematically illustrates an exemplary head mountable device 2 comprising a first mirror 18. The first mirror 18 is configured for mirroring images 9 of the first eye 20 towards the camera system 6. The first mirror 18 may be configured for mirroring images 9 of the first eye 20 towards a first camera of the camera system 6. Alternatively or additionally, the first mirror 18 is configured for mirroring images 27 of the second eye 22 towards the camera system 6. The first mirror 18 may be configured for mirroring images 27 of the second eye 22 towards the first camera and/or a second camera of the camera system 6.

The head mountable device 2 may further comprise a second mirror (not shown), wherein the second mirror is configured for mirroring images 27 of the second eye 22 towards the camera system 6. The second mirror may be configured for mirroring images 27 of the second eye 22 towards the first camera and/or the second camera of the camera system 6.

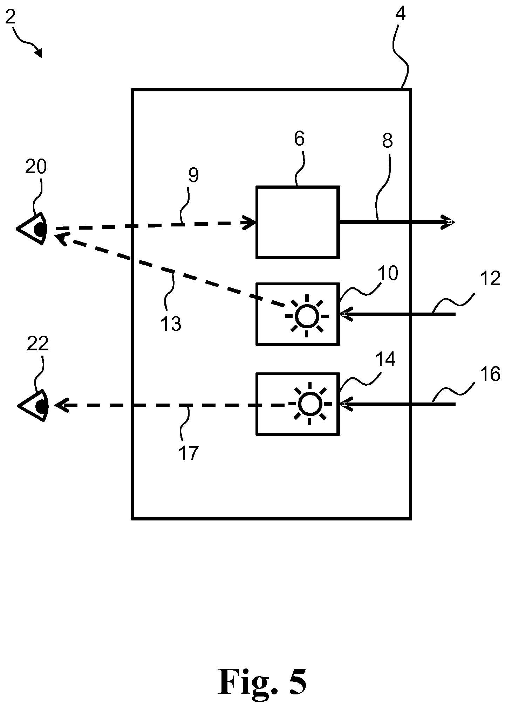

Fig. 5 schematically illustrates an exemplary head mountable device 2 for measuring eye movement. The head mountable device 2 comprises a frame 4, a camera system 6, a first liquid crystal display (LCD) shutter 10, and a second liquid crystal display (LCD) shutter 14. In the depicted example, the camera system 6, the first LCD shutter 10, and the second LCD shutter 14 are mounted on the frame 4.

The camera system 6 is configured to obtain a first set of images 8 of a first eye 20 of a user. The camera system 6 detects images 9 of the first eye 20 and converts the images 9 of the first eye 20 to the first set of images 8 of the first eye 20.

The first LCD shutter 10 is configured to control passage of light 13 to at least part of the first eye 20 based on a first control signal 12. The first LCD shutter 10 is configured to operate in a first primary mode and a first secondary mode. Passage of light 13 through the first LCD shutter 10 in the first secondary mode is restricted relative to the first primary mode.

The second LCD shutter 14 is configured to control passage of light 17 to at least part of the second eye 22 based on a second control signal 16. The second LCD shutter 14 is configured to operate in a second primary mode and a second secondary mode. Passage of light 17 through the second LCD shutter 14 in the second secondary mode is restricted relative to the second primary mode.

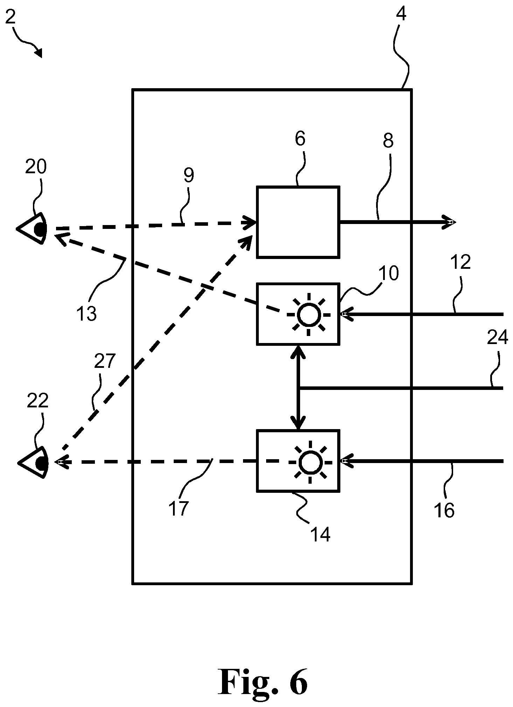

Fig. 6 schematically illustrates an exemplary head mountable device 2 comprising the same features as the exemplary head mountable device as depicted in Fig. 5. However, in Fig. 6, the first LCD shutter 10 is configured to control passage of light 13 to at least part of the first eye 20 based on the first control signal 12 and a common control signal 24. Furthermore, the second LCD shutter 14 is configured to control passage of light 17 to at least part of the second eye 22 based on the second control signal 16 and the common control signal 24. The first control signal 12 and/or the second control signal 16 and/or the common control signal 24 may be alternating current (AC) signals and/or bipolar square wave voltage signals. The first LCD shutter 10 may be configured to operate in the first primary mode when the first control signal 12 and the common control signal 24 are in phase. Conversely, the first LCD shutter 10 may be configured to operate in the first secondary mode when the first control signal 12 and the common control signal 24 are out of phase, e.g. when the first control signal 12 and the common control signal 24 are 180 degrees out of phase. The second LCD shutter 14 may be configured to operate in the second primary mode when the second control signal 16 and the common control signal 24 are in phase. Conversely, the second LCD shutter 14 may be configured to operate in the second secondary mode when the second control signal 16 and the common control signal 24 are out of phase, e.g. when the second control signal 16 and the common control signal 24 are 180 degrees out of phase.

Furthermore, Fig. 6 illustrates an exemplary head mountable device 2, wherein the camera system 6 is configured to obtain a second set of images 26 of a second eye of the user. The camera system 6 is configured to obtain a first set of images 8 of a first eye 20 of a user, and the camera system 6 is configured to obtain a second set of images 26 of a second eye 22 of the user. The camera system 6 detects images 9 of the first eye 20 and converts the images 9 of the first eye 20 to the first set of images 8 of the first eye 20. The camera system 6 detects images 27 of the second eye 22 and converts the images 27 of the second eye 22 to the second set of images 26 of the second eye 22.

Fig. 7 schematically illustrates an exemplary camera system 6 for a head mountable device 2. The camera system 6 comprises a first camera 40. The first camera 40 detects images 9 of a first eye 20 of a user and converts the images 9 of the first eye 20 to a first set of images 8 of the first eye 20. The first camera 40 converts the images 9 of the first eye 20 to the first set of images 8 of the first eye 20 with a first frame rate and a first resolution. Alternatively and/or additionally, the first camera 40 detects images 27 of a second eye 22 of the user and converts the images 27 of the second eye 22 to a second set of images 26 of the second eye 22. The first camera 40 converts the images 27 of the second eye 22 to a second set of images 26 of the second eye 22 with a second frame rate and a second resolution.

Fig. 8 schematically illustrates an exemplary camera system 6 for a head mountable device 2. The camera system 6 of Fig. 8 comprises a first camera 40 and a second camera 42. The first camera 40 detects images 9 of a first eye 20 and converts the images 9 of the first eye 20 to a first set of images 8 of the first eye 20. The first camera converts the images 9 of the first eye 20 to a first set of images 8 of the first eye 20 with a first frame rate and a first resolution. The second camera 42 detects images 27 of a second eye 22 and converts the images 27 of the second eye 22 to a second set of images 26 of the second eye 22. The second camera 42 converts the images 27 of the second eye 22 to a second set of images 26 of the second eye 22 with a second frame rate and a second resolution.

In relation to any of Figs. 7 and 8, the first camera 40 and/or the second camera 42 may be adapted to enable detection of eye saccades of the first eye 20 and/or second eye 30. For example, the first frame rate and/or the second frame rate may be higher than 125 fps. The first camera 40 and/or the second camera 42 may be able to detect electromagnetic radiation such as infrared radiation (IR), laser light, and/or colored visible light, e.g. red, blue, green, and/or orange visible light. The first camera 40 and/or the second camera 42 may be able to detect electromagnetic radiation of a first light source (not shown).

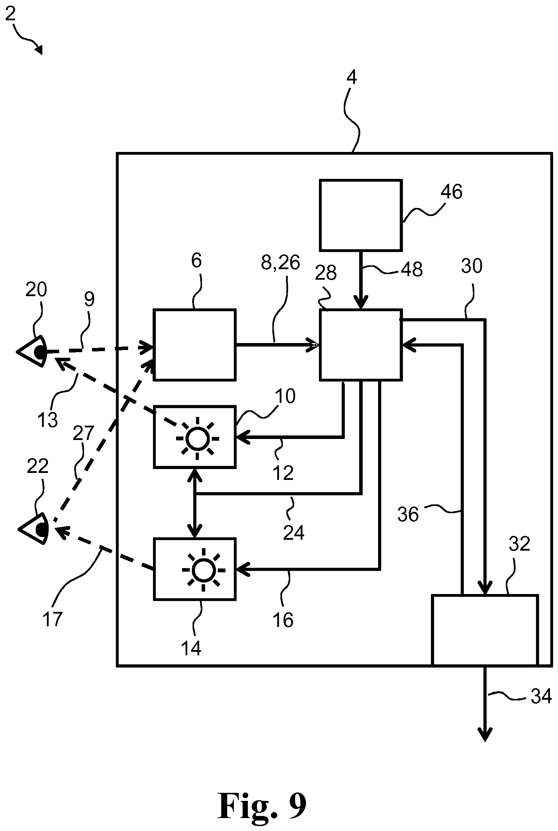

Fig. 9 schematically illustrates an exemplary head mountable device 2, further comprising a number of additional features, which individually and/or in combination may be added to the head mountable device 2 described in relation to any of the preceding figures. The head mountable device 2 of Fig. 9 further comprises a processing unit 28, an interface 32, and a motion sensor 46.

The frame 4 comprises the camera system 6, the first LCD shutter 10, the second LCD shutter 14, the processing unit 28, the interface 32, and the first motion sensor 46. In other exemplary head mountable devices (not shown), the frame 4 may comprise one or more of the camera system 6, the first LCD shutter 10, the second LCD shutter 14, the processing unit 28, the interface 32, and the first motion sensor 46.

The processing unit 28 may be configured to process the first set of images 8 and/or the second set of images 26 and provide a processing unit output 30. The processing unit output 30 may be based on the first set of images 8 and/or the processing unit output 30 may be based on the second set of images 26. Furthermore, the first control signal 12 and the second control signal 16 and the common control signal 24 is controlled by the processing unit 28. In alternative exemplary head mountable devices, the processing unit 28 may control one or more of the first control signal 12 and the second control signal 16 and the common control signal 24.

The interface 32 provides a device output 34. The device output 34 may be based on the first set of images 8 and/or the second set of images 26. In the depicted example, the device output 34 is based on the processing unit output 30 which is based on the first set of images 8 and/or the second set of images 26.

In the depicted example, the interface 32 provides a processing unit control signal 36. However, in other exemplary head mountable devices, the provision of a processing unit control signal 36 may be omitted. The processing unit control signal 36 may allow user control of the processing unit 28 and/or the head mountable device 2 via an input device, such as a user interface, of the interface 32.

The motion sensor 46 is configured to detect movement of the head mountable device 2. The processing unit 28 is connected to the motion sensor 46. The motion sensor 46 provides a sensor output 48. The processing unit 28 is configured to process the sensor output 48 from the first motion sensor 46, and the processing unit output 30 may be based on the sensor output 48. The motion sensor 46 may comprise one or more gyroscope(s) and/or one or more accelerometer(s).

The processing unit 28 may compress and/or reduce the amount of data in the processing unit output 30. For example, in order for the interface 32 to transmit the device output 34, or a part of the device output 34, wirelessly, without substantial delay e.g. a delay of the order of 10 ms, the processing unit output 30 may be compressed and/or reduced. For example, the processing unit output 30 may comprise a first secondary set of images with a first secondary frame rate and a first secondary resolution, wherein the first secondary frame rate is smaller than the first frame rate and/or the first secondary resolution is smaller than the first resolution. Alternatively and/or additionally the processing unit output 30 may comprise a second secondary set of images with a second secondary frame rate and a second secondary resolution, wherein the second secondary frame rate is smaller than the second frame rate and/or the second secondary resolution is smaller than the second resolution.

Additionally and/or alternatively, the processing unit 28 may be configured to compress an initial processing unit output based on the first set of images 8 and/or the second set of images 26, wherein the size of the processing unit output 30 is below 20%, such as 10%, such as 5% of the size of the initial processing unit output.

The processing unit output 30 and/or the device output 34 may be indicative of one or more parameters of the user, such as an ophthalmologic parameter, a vestibular parameter, and/or a neurologic parameter.

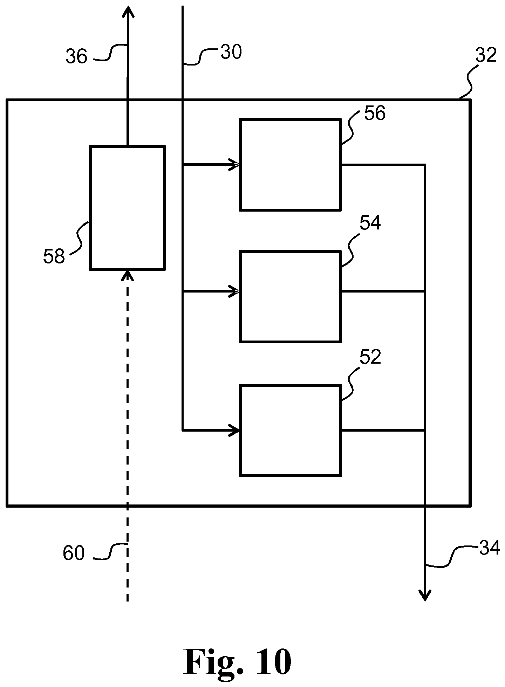

Fig. 10 schematically illustrates an exemplary interface 32. The interface 32 comprises a wireless transmitter unit 52, a first display 54, a speaker 56, and/or an input device 58. The interface 32 may in alternative configurations (not shown) comprise one or more of the wireless transmitter unit 52, the first display 54, the speaker 56 and the input device 58.

The wireless transmitter unit 52 receives the processing unit output 30, or part of the processing unit output 30, and transmits the device output 34, or a part of the device output 34, wirelessly to a wireless receiver (not shown). The wireless transmitter unit 52 may be a Bluetooth transmitter, a WiFi transmitter, a 3G transmitter and/or a 4G transmitter. The wireless transmitter unit 52 may further be configured to transmit the device output 34, or a part of the device output 34, with a low latency to enable live preview of the device output 34 in an external display. The latency may be less than 40 ms such as less than 20 ms such as less than 10 ms.

The first display 54 receives the processing unit output 30, or part of the processing unit output 30, and visually presents the device output 34, or a part of the device output 34, to a user or an operator of the device. The first display 54 may be an organic light emitting diode (OLED), an OLED display, a light emitting diode (LED), an LED display, and/or an e-ink display.

The speaker 56 receives the processing unit output 30, or part of the processing unit output 30, and audiologically, by means of sounds, presents the device output 34, or a part of the device output 34, to a user or an operator of the device.

The input device 58 enables control of the head mountable device 2. User interaction 60 is detected by the input device 58, and the input device 58 provides a control signal 36 to the processing unit 12. The input device 58 may comprise a push button, a switch, and/or a touch display.

The device output 34 may be indicative of a positive/negative result of a test. For example, the device output 34 may comprise lighting up the first display 54 in a red colour if the test result is negative, and/or lighting up the first display 54 in a green colour if the test result is positive. For example, the device output 34 is indicative of an ophthalmologic parameter of the user, the device output 34 is indicative of a vestibular parameter of the user, and/or the device output 34 is indicative of a neurologic parameter of the user.

The device output 34 may comprise a plurality of output images based on the first set of images 8 and/or based on the second set of images 26. For example, the device output 34 may provide a live preview of the images 9, 27 of the first eye 20 and/or the second eye 22. The live preview may be transmitted wirelessly via the wireless transmitter 52 to an external display, e.g. a display of an external device, such as a tablet computer, a smart phone, or a laptop.

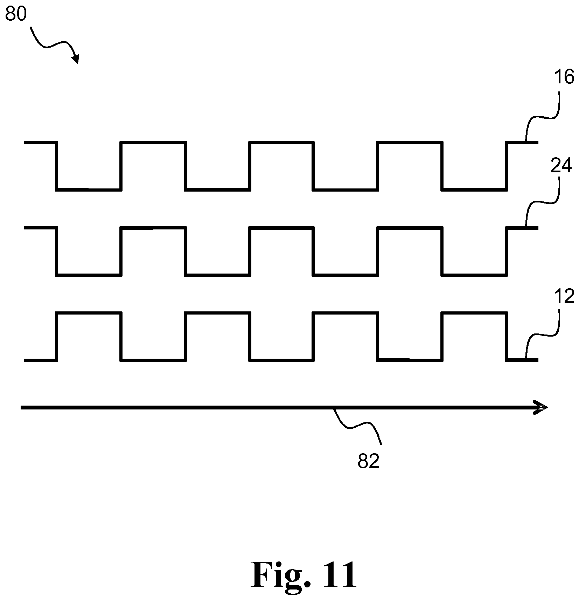

Fig. 11 illustrates voltage traces 80 of exemplary control signals 12, 16, 24 for a head mountable device 2. The voltage traces 80 of the control signals 12, 16, 24 are shown on a time axis 82. In the depicted example, the control signals 12, 16, 24 are bipolar square wave signals. The control signals shown are the first control signal 12 for the first LCD shutter 10, the second control signal 16 for the second LCD shutter 14, and the common control signal 24 for both the first LCD shutter 10 and second LCD shutter 14.

The depicted example shows the first control signal 12 and the common control signal 24 to be 180 degrees out of phase. This generates a voltage difference between the first control signal 12 and the common control signal 24. The voltage difference may result in the first LCD shutter 10 to operate in the first secondary operating mode where passage of light through the first LCD shutter 10 is restricted and/or blocked.

The depicted example shows the second control signal 16 and the common control signal 24 to be in phase. This generates no voltage difference between the second control signal 16 and the common control signal 24. The zero voltage difference may result in the second LCD shutter 14 to operate in the second primary operating mode where passage of light through the second LCD shutter 14 is not restricted and/or blocked, i.e. passage of light through the second LCD shutter 14 is allowed.

Changing the operating modes of the first LCD shutter 10 and/or the second LCD shutter 14 may be achieved by altering the first control signal 12, the common control signal 24, and/or the second control signal 16 to be in phase or 180 degrees out of phase.

In alternative embodiments (not shown), the first control signal 12 and/or the second control signal 16 may be DC signals. However, using AC signals and/or bipolar square wave signals, possible migration of crystals within the LCD shutters 10, 14 will be prevented.

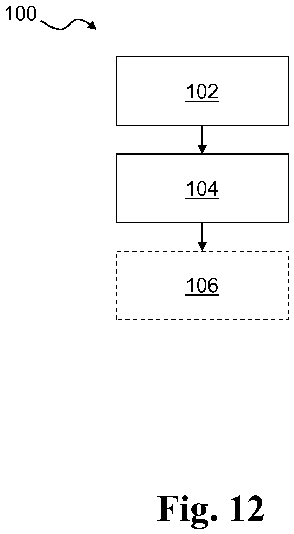

Fig. 12 shows a flow diagram of a method 100 for measuring eye movement. The method 100 may comprise using a head mountable device 2, such as a head mountable device 2 as described in relation to any of the previous figures. The method comprises adjusting 102 passage of light to at least part of a first eye and/or a second eye, and obtaining 104 a first set of images of the first eye and/or a second set of images of the second eye. Optionally, the method may comprise providing 106 a device output based on the first set of images and/or the second set of images.

Adjusting 102 passage of light to at least part of the first eye and/or a second eye may be achieved by operation of the first LCD shutter 10 and/or second LCD shutter 14 of the head mountable device 2.

Obtaining 104 the first set of images and/or the second set of images may be achieved by the camera system 6 of the head mountable device 2. The first set of images and/or the second set on images may be obtained with a respective first frame rate and/or second frame rate enabling detection of eye saccades of the respective first eye and/or second eye, e.g. a first frame rate and/or a second frame rate higher than 125 fps.

The device output provided 106 may be indicative of one or more parameters of the user, e.g. a vestibular parameter of the user, an ophthalmologic parameter of the user, and/or a neurologic parameter of the user. The device output may further be indicative of a test result, such as a vestibular test, an ophthalmologic test and/or a neurologic test. The device output may be provided 106 via an audiologic output, a visual output, and/or wireless transmission to an external device.

The method 100 may furthermore comprise mounting (not shown) the head mountable device 2 to a head of the user, and/or detecting (not shown) movement of the head mountable device 2.

Mounting of the head mountable device 2 to a head of the user may be performed by an operator, and may involve fastening the head mountable device 2 to the head of the user to avoid movement of the head mountable device 2 relative to the head of the user. If the device is tightly fixed to the head, moving the head of the user involves movement of the head mountable device 2. Thus, the movement of the device 2 corresponds to the movement of the head of the user. Detecting of the movement of the head mountable device is therefore indicative of the moving of the head of the user. Although particular features have been shown and described, it will be understood that they are not intended to limit the claimed invention, and it will be made obvious to those skilled in the art that various changes and modifications may be made without departing from the scope of the appended claims.

### LIST OF REFERENCES

- 2: head mountable device
- 4: frame
- 6: camera system
- 8: first set of images
- 9: image(s) of first eye
- 10: first LCD shutter
- 12: first control signal
- 13: light through first LCD shutter
- 14: second LCD shutter
- 16: second control signal
- 17: light through second LCD shutter
- 18: first mirror
- 20: first eye
- 22: second eye
- 24: common control signal
- 26: second set of images
- 27: image(s) of second eye
- 28: processing unit
- 30: processing unit output
- 32: interface
- 34: device output
- 36: processing unit control signal
- 40: first camera
- 42: second camera
- 46: motion sensor
- 48: sensor output
- 52: wireless transmitter unit
- 54: first display
- 56: speaker
- 58: input device
- 60: user interaction
- 80: control signals
- 82: time axis
- 100: method
- 102: adjusting passage of light
- 104: obtaining first set of images
- 106: providing device output

## Claims

1. A head mountable device (2) for measuring eye movement, the head mountable device comprising:
- a frame (4);
- a camera system (6) comprising a first camera (40), wherein the camera system (6) is configured to obtain a first set of images (8) of a first eye of a user, wherein the first set of images (8) is configured to be obtained with a first frame rate, wherein the first frame rate is selected such as to enable detection of eye saccades of the first eye;
a first liquid crystal display (LCD) shutter (10) configured to control passage of light to at least part of the first eye (20) based on a first control signal (12), the first LCD shutter (10) is configured to operate in a first primary mode and a first secondary mode, where passage of light through the first LCD shutter (10) in the first secondary mode is restricted relative to the first primary mode, wherein the head mountable device (2) further comprises a second liquid crystal display (LCD) shutter (14) configured to control passage of light to at least part of a second eye (22) of the user based on a second control signal (16), the second LCD shutter (14) is configured to operate in a second primary mode and a second secondary mode, where passage of light through the second LCD shutter (14) in the second secondary mode is restricted relative to the second primary mode, **characterized in that** the first LCD shutter (10) is configured to control passage of light through the first LCD shutter (10) based on the first control signal (12) and a common control signal (24) and **in that** the second LCD shutter (14) is configured to control passage of light through the second LCD shutter (14) based on the second control signal (16) and said common control signal (24), where the first control signal (12), the second control signal (16) and the common control signal (24) are voltage signals.

2. Head mountable device according to claim 1, wherein the head mountable device (2) comprises a first mirror (18) for mirroring images of the first eye (20) towards the first camera (40).

3. Head mountable device (2) according to any of claims 1-2, wherein the first control signal (12), the second control signal (16), and/or the common control signal (24) are alternating current (AC) signals and/or bipolar square wave voltage signals.

4. Head mountable device (2) according to any of the preceding claims, wherein voltage of the first control signal (12), the second control signal (16), and/or the common control signal (24) is in the range from 2 to 14 volts, such as 5 volts or such as 10 volts.

5. Head mountable device (2) according to any of the preceding claims, wherein the camera system (6) is configured to obtain a second set of images (26) of a second eye (22) of the user.

6. Head mountable device (2) according to claim 5, wherein the camera system (6) comprises a second camera (42) configured to obtain the second set of images (26).

7. Head mountable device (2) according to any of the preceding claims, wherein the second set of images (26) is configured to be obtained with a second frame rate, wherein the second frame rate is selected such as to enable detection of eye saccades of the second eye (22).

8. Head mountable device (2) according to any of the preceding claims, wherein the head mountable device (2) comprises a processing unit (28) configured to process the first set of images (8) and providing a processing unit output (30) based on the first set of images (8).

9. Head mountable device (2) according to claim 8, wherein the first control signal (12), the second control signal (16), and/or the common control signal (24) are controlled by the processing unit (28).

10. Head mountable device (2) according to any of the preceding claims, wherein the head mountable device (2) comprises an interface (32) for providing a device output (34) based on the first set of images (8).

11. Head mountable device (2) according to any of the preceding claims, wherein the frame (4) accommodates the camera system (6) and the first LCD shutter (10).

12. Method for measuring eye movement of a user using a head mountable device (2) comprising a frame (4), a camera system (6) comprising a first camera (40), and a first liquid crystal display (LCD) shutter (10) configured to control passage of light to at least part of a first eye (20) of a user based on a first control signal (12), the first LCD shutter (10) is configured to operate in a first primary mode and a first secondary mode, where passage of light through the first LCD shutter (10) in the first secondary mode is restricted relative to the first primary mode, wherein the head mountable device (2) further comprises a second liquid crystal display (LCD) shutter (14) configured to control passage of light to at least part of a second eye (22) of the user based on a second control signal (16), the second LCD shutter (14) is configured to operate in a second primary mode and a second secondary mode, where passage of light through the second LCD shutter (14) in the second secondary mode is restricted relative to the second primary mode, the method comprising:
- adjusting (102) passage of light to at least part of the first eye (20) by operation of the first LCD shutter (10);
- obtaining (104) a first set of images (8) of the first eye (20) by the camera system (6), wherein the first set of images (8) is configured to be obtained with a first frame rate, wherein the first frame rate is selected such as to enable detection of eye saccades of the first eye;
- adjusting (102) passage of light to at least part of the second eye (22) by operation of the second LCD shutter (14);
- obtaining (104) a second set of images (26) of the second eye (22) by the camera system (6);
- configuring the first LCD shutter (10) to control passage of light through the first LCD shutter (10) based on the first control signal (12) and a common control signal (24); and
- configuring the second LCD shutter (14) to control passage of light through the second LCD shutter (14) based on the second control signal (16) and said common control signal (24), where the first control signal (12), the second control signal (16) and the common control signal (24) are voltage signals.

13. Method according to claim 12, wherein the method comprises providing a device output (34) based on the first set of images (8).

14. Method according to claim 12, wherein the method comprises providing a device output (34) based on the first set of images (8) and on the second set of images (26).

## Patentansprüche

1. Am Kopf montierbare Vorrichtung (2) zur Messung einer Augenbewegung, wobei die am Kopf montierbare Vorrichtung umfasst:
- einen Rahmen (4);
- ein Kamerasystem (6) umfassend eine erste Kamera (40), wobei das Kamerasystem (6) ausgelegt ist, um einen ersten Satz von Bildern (8) eines ersten Auges eines Benutzers zu erhalten, wobei der erste Satz von Bildern (8) ausgelegt ist, mit einer ersten Rahmengeschwindigkeit erhalten zu werden, wobei die erste Rahmengeschwindigkeit derart ausgewählt ist, dass sie die Erkennung von Augensakkaden des ersten Auges ermöglicht;
einen ersten Flüssigkristallanzeige- (LCD-) Shutter (10), der ausgelegt ist, um den Durchgang von Licht zu mindestens einem Teil des ersten Auges (20) basierend auf einem ersten Steuersignal (12) zu steuern, der erste LCD-Shutter (10) ausgelegt ist, um in einer ersten primären Betriebsart und einer ersten sekundären Betriebsart zu arbeiten, wobei der Durchgang von Licht durch den ersten LCD-Shutter (10) in der ersten sekundären Betriebsart relativ zur ersten primären Betriebsart beschränkt ist, wobei die am Kopf montierbare Vorrichtung (2) ferner einen zweiten Flüssigkristallanzeige- (LCD-) Shutter (14) umfasst, der ausgelegt ist, um einen Durchgang von Licht zu mindestens einem Teil eines zweiten Auges (22) der Benutzers basierend auf einem zweiten Steuersignal (16) zu steuern, der erste LCD-Shutter (14) ausgelegt ist, um in einer zweiten primären Betriebsart und einer zweiten sekundären Betriebsart zu arbeiten, wobei der Durchgang von Licht durch den zweiten LCD-Shutter (14) in der zweiten sekundären Betriebsart relativ zur zweiten primären Betriebsart beschränkt ist, **dadurch gekennzeichnet, dass** der erste LCD-Shutter (10) ausgelegt ist, um einen Durchgang von Licht durch den ersten LCD-Shutter (10) basierend auf dem ersten Steuersignal (12) und einem gemeinsamen Steuersignal (24) zu steuern, und **dass** der zweite LCD-Shutter (14) ausgelegt ist, um einen Durchgang von Licht durch den zweiten LCD-Shutter (14) basierend auf dem zweiten Steuersignal (16) und dem gemeinsamen Steuersignal (24) zu steuern, wobei das erste Steuersignal (12), das zweite Steuersignal (16) und das gemeinsame Steuersignal (24) Spannungssignale sind.

2. Am Kopf montierbare Vorrichtung nach Anspruch 1, wobei die am Kopf montierbare Vorrichtung (2) einen ersten Spiegel (18) zur Spiegelung von Bildern des ersten Auges (20) in Richtung auf die erste Kamera (40) zu umfasst.

3. Am Kopf montierbare Vorrichtung (2) nach einem der Ansprüche 1-2, wobei das erste Steuersignal (12), das zweite Steuersignal (16), und/oder das gemeinsame Steuersignal (24) Wechselstrom- (AC-) Signale und/oder bipolare Rechteckspannungssignale sind.

4. Am Kopf montierbare Vorrichtung (2) nach einem der vorgehenden Ansprüche, wobei die Spannung des ersten Steuersignals (12), des zweiten Steuersignals (16) und/oder des gemeinsamen Steuersignals (24) im Bereich von 2 bis 14 Volt, wie beispielsweise 5 Volt oder wie beispielsweise 10 Volt, liegt.

5. Am Kopf montierbare Vorrichtung (2) nach einem der vorgehenden Ansprüche, wobei das Kamerasystem (6) ausgelegt ist, um einen zweiten Satz von Bildern (26) eines zweiten Auges (22) des Benutzers zu erhalten.

6. Am Kopf montierbare Vorrichtung (2) nach Anspruch 5, wobei das Kamerasystem (6) eine zweite Kamera (42) umfasst, die ausgelegt ist, um einen zweiten Satz von Bildern (26) zu erhalten.

7. Am Kopf montierbare Vorrichtung (2) nach einem der vorgehenden Ansprüche, wobei der zweite Satz von Bildern (26) ausgelegt ist, um mit einer zweiten Rahmengeschwindigkeit erhalten zu werden, wobei die zweite Rahmengeschwindigkeit derart ausgewählt ist, dass sie eine Erkennung von Augensackaden des zweiten Auges (22) ermöglicht.

8. Am Kopf montierbare Vorrichtung (2) nach einem der vorgehenden Ansprüche, wobei die am Kopf montierbare Vorrichtung (2) eine Verarbeitungseinheit (28) umfasst, die ausgelegt ist, um den ersten Satz von Bildern (8) zu verarbeiten und eine Verarbeitungseinheitausgabe (30) basierend auf dem ersten Satz von Bildern (8) bereitzustellen.

9. Am Kopf montierbare Vorrichtung (2) nach Anspruch 8, wobei das erste Steuersignal (12), das zweite Steuersignal (16), und/oder das gemeinsame Steuersignal (24) durch die Verarbeitungseinheit (28) gesteuert sind.

10. Am Kopf montierbare Vorrichtung (2) nach einem der vorgehenden Ansprüche, wobei die am Kopf montierbare Vorrichtung (2) eine Schnittstelle (32) zum Bereitstellen einer Vorrichtungsausgabe (34) basierend auf dem ersten Satz von Bildern (8) umfasst.

11. Am Kopf montierbare Vorrichtung (2) nach einem der vorgehenden Ansprüche, wobei der Rahmen (4) das Kamerasystem (6) und den ersten LCD-Shutter (10) aufnimmt.

12. Verfahren zur Messung einer Augenbewegung eines Benutzers, der eine am Kopf montierbare Vorrichtung (2) benutzt, die einen Rahmen (4), ein Kamerasystem (6) umfassend eine erste Kamera (40) und einen ersten Flüssigkeitskristallanzeige- (LCD-) Shutter (10) umfasst, der ausgelegt ist, um den Durchgang von Licht zu mindestens einem Teil eines ersten Auges (20) eines Benutzers basierend auf einem ersten Steuersignal (12) zu steuern, wobei der erste LCD-Shutter (10) ausgelegt ist, um in einer ersten primären Betriebsart und einer ersten sekundären Betriebsart zu arbeiten, wobei der Durchgang von Licht durch den ersten LCD-Shutter (10) in der ersten sekundären Betriebsart relativ zur ersten primären Betriebsart beschränkt ist, wobei die am Kopf montierbare Vorrichtung (2) ferner einen zweiten Flüssigkristallanzeige-(LCD-) Shutter (14) umfasst, der ausgelegt ist, um den Durchgang von Licht zu mindestens einem Teil eines zweiten Auges (22) des Benutzers basierend auf einem zweiten Steuersignal (16) zu steuern, wobei der zweite LCD-Shutter (14) ausgelegt ist, um in einer zweiten primären Betriebsart und einer zweiten sekundären Betriebsart zu arbeiten, wobei der Durchgang von Licht durch den zweiten LCD-Shutter (14) in der zweiten sekundären Betriebsart relativ zur zweiten primären Betriebsart beschränkt ist, wobei das Verfahren Folgendes umfasst:
- Anpassen (102) des Durchgangs von Licht an mindestens einen Teil des ersten Auges (20) bei Betätigung des ersten LCD-Shutters (10);
- Erhalten (104) eines durch das Kamerasystem (6) erhaltenen ersten Satzes von Bildern (8) des ersten Auges (20), wobei der erste Satz von Bildern (8) ausgelegt ist, um mit einer ersten Rahmengeschwindigkeit erhalten zu werden, wobei die erste Rahmengeschwindigkeit derart ausgewählt ist, dass sie die Erkennung von Augensakkaden des ersten Auges ermöglicht;
- Anpassen (102) des Durchgangs von Licht an mindestens einen Teil des zweiten Auges (22) bei Betätigung des zweiten LCD-Shutters (14);
- Erhalten (104) eines zweiten Satzes von Bildern (26) des zweiten Auges (22) durch das Kamerasystem (6);
- Konfigurieren des ersten LCD-Shutters (10), um den Durchgang von Licht durch den ersten LCD-Shutter (10) basierend auf dem ersten Steuersignal (12) und einem gemeinsamen Steuersignal (24) zu steuern; und
- Konfigurieren des zweiten LCD-Shutters (14), um einen Durchgang von Licht durch den zweiten LCD-Shutter (14) basierend auf dem zweiten Steuersignal (16) und dem gemeinsamen Steuersignal (24) zu steuern, wobei das erste Steuersignal (12), das zweite Steuersignal (16) und das gemeinsame Steuersignal (24) Spannungssignale sind.

13. Verfahren nach Anspruch 12, wobei das Verfahren das Bereitstellen einer Vorrichtungsausgabe (34) basierend auf dem ersten Satz von Bildern (8) umfasst.

14. Verfahren nach Anspruch 12, wobei das Verfahren das Bereitstellen einer Vorrichtungsausgabe (34) basierend auf dem ersten Satz von Bildern (8) und auf dem zweiten Satz von Bildern (26) umfasst.

## Revendications

1. Dispositif pouvant être monté sur la tête (2) pour mesurer le mouvement oculaire, le dispositif pouvant être monté sur la tête comprenant :
- un cadre (4) ;
- un système de caméra (6) comprenant une première caméra (40), le système de caméra (6) étant configuré pour obtenir un premier ensemble d'images (8) d'un premier œil d'un utilisateur, le premier ensemble d'images (8) étant configuré pour être obtenu avec une première fréquence d'images, la première fréquence d'images étant sélectionnée de manière à permettre la détection des saccades oculaires du premier oeil ;
un premier obturateur (10) d'affichage à cristaux liquides (LCD) configuré pour commander le passage de la lumière vers au moins une partie du premier œil (20) sur la base d'un premier signal de commande (12), le premier obturateur LCD (10) étant configuré pour fonctionner dans un premier mode primaire et un premier mode secondaire, où le passage de la lumière à travers le premier obturateur LCD (10) dans le premier mode secondaire est limité par rapport au premier mode primaire, le dispositif pouvant être monté sur la tête (2) comprenant en outre un deuxième obturateur (14) d'affichage à cristaux liquides (LCD) configuré pour commander le passage de la lumière vers au moins une partie d'un deuxième œil (22) de l'utilisateur sur la base d'un deuxième signal de commande (16), le deuxième obturateur LCD (14) étant configuré pour fonctionner dans un deuxième mode primaire et un deuxième mode secondaire, où le passage de la lumière à travers le deuxième obturateur LCD (14) dans le deuxième mode secondaire est limité par rapport au deuxième mode primaire, **caractérisé en ce que** le premier obturateur LCD (10) est configuré pour commander le passage de la lumière à travers le premier obturateur (10) sur la base du premier signal de commande (12) et d'un signal de commande commun (24), et **en ce que** le deuxième obturateur LCD (14) est configuré pour commander le passage de la lumière à travers le deuxième obturateur (14) sur la base du second signal de commande (16) et du signal de commande commun (24), le premier signal de commande (12), le deuxième signal de commande (16) et / ou le signal de commande commun (24) sont des signaux de tension.

2. Dispositif pouvant être monté sur la tête selon la revendication 1, dans lequel le dispositif pouvant être monté sur la tête (2) comprend un premier miroir (18) pour refléter les images du premier œil (20) vers la première caméra (40).

3. Dispositif pouvant être monté sur la tête (2) selon l'une quelconque des revendications 1 et 2, dans lequel le premier signal de commande (12), le deuxième signal de commande (16) et / ou le signal de commande commun (24) sont des signaux de courant alternatif (AC) et / ou des signaux de tension bipolaire à tension carrée.

4. Dispositif pouvant être monté sur la tête (2) selon l'une quelconque des revendications précédentes, dans lequel la tension du premier signal de commande (12), du deuxième signal de commande (16) et / ou du signal de commande commun (24) est dans la plage de 2 à 14 volts, telle que 5 volts ou telle que 10 volts.

5. Dispositif pouvant être monté sur la tête (2) selon l'une quelconque des revendications précédentes, dans lequel le système de caméra (6) est configuré pour obtenir un deuxième ensemble d'images (26) d'un deuxième œil (22) de l'utilisateur.

6. Dispositif pouvant être monté sur la tête (2) selon la revendication 5, dans lequel le système de caméra (6) comprend une deuxième caméra (42) configurée pour obtenir le deuxième ensemble d'images (26).

7. Dispositif pouvant être monté sur la tête (2) selon l'une quelconque des revendications précédentes, dans lequel le deuxième ensemble d'images (26) est configuré pour être obtenu avec une deuxième fréquence d'images, la deuxième fréquence d'images étant sélectionnée de manière à permettre la détection des saccades oculaires du deuxième œil (22).

8. Dispositif pouvant être monté sur la tête (2) selon l'une quelconque des revendications précédentes, dans lequel le dispositif pouvant être monté sur la tête (2) comprend une unité de traitement (28) configurée pour traiter le premier ensemble d'images (8) et fournir une sortie d'unité de traitement (30) sur la base du premier ensemble d'images (8).

9. Dispositif pouvant être monté sur la tête (2) selon la revendication 8, dans lequel le premier signal de commande (12), le deuxième signal de commande (16) et / ou le signal de commande commun (24) sont commandés par l'unité de traitement (28).

10. Dispositif pouvant être monté sur la tête (2) selon l'une quelconque des revendications précédentes, dans lequel le dispositif pouvant être monté sur la tête (2) comprend une interface (32) pour fournir une sortie de dispositif (34) sur la base du premier ensemble d'images (8).

11. Dispositif pouvant être monté sur la tête (2) selon l'une quelconque des revendications précédentes, dans lequel le cadre (4) reçoit le système de caméra (6) et le premier obturateur (10).

12. Procédé de mesure des mouvements oculaires d'un utilisateur utilisant un dispositif pouvant être monté sur la tête (2) comprenant un cadre (4), un système de caméra (6) comprenant une première caméra (40), et un premier obturateur (10) d'affichage à cristaux liquides (LCD) configuré pour commander le passage de la lumière vers au moins une partie d'un premier œil (20) d'un utilisateur sur la base d'un premier signal de commande (12), le premier obturateur LCD (10) étant configuré pour fonctionner dans un premier mode primaire et un premier mode secondaire, où le passage de la lumière à travers le premier obturateur LCD (10) dans le premier mode secondaire est limité par rapport au premier mode primaire, le dispositif pouvant être monté sur la tête (2) comprenant en outre un deuxième obturateur (14) d'affichage à cristaux liquides (LCD) configuré pour commander le passage de la lumière vers au moins une partie d'un deuxième œil (22) sur la base d'un deuxième signal de commande (16), le deuxième obturateur LCD (14) étant configuré pour fonctionner dans un deuxième mode primaire et un deuxième mode secondaire, où le passage de la lumière à travers le deuxième obturateur LCD (14) dans le deuxième mode secondaire est limité par rapport au deuxième mode primaire, le procédé comprenant les étapes consistant à :
- ajuster (102) le passage de la lumière sur au moins une partie du premier œil (20) en actionnant le premier obturateur LCD (10) ;
- obtenir (104) un premier ensemble d'images (8) du premier œil (20) par le système de caméra (6), le premier ensemble d'images (8) étant configuré pour être obtenu avec une première fréquence d'images, la première fréquence d'images étant sélectionnée de manière à permettre la détection des saccades oculaires du premier œil ;
- ajuster (102) le passage de la lumière sur au moins une partie du deuxième œil (22) en actionnant le deuxième obturateur LCD (14) ;
- obtenir (104) un deuxième ensemble d'images (26) du deuxième œil (22) par le système de caméra (6) ;
- configurer le premier obturateur LCD (10) pour commander le passage de la lumière à travers le premier obturateur (10) sur la base du premier signal de commande (12) et d'un signal de commande commun (24) ; et
- configurer le deuxième obturateur LCD (14) pour commander le passage de la lumière à travers le deuxième obturateur (14) sur la base du deuxième signal de commande (16) et du signal de commande commun (24), le premier signal de commande (12), le deuxième signal de commande (16) et / ou le signal de commande commun (24) sont des signaux de tension.

13. Procédé selon la revendication 12, dans lequel le procédé comprend la fourniture d'une sortie de dispositif (34) basée sur le premier ensemble d'images (8).

14. Procédé selon la revendication 12, dans lequel le procédé comprend la fourniture d'une sortie de dispositif (34) basée sur le premier ensemble d'images (8) et sur le deuxième ensemble d'images (26).
